# EUROPEAN PATENT APPLICATION

(11) **EP 4 837 562 A1**
(43) Date of publication of application: **11.11.2026**
(21) Application number: 25305652.7
(22) Date of filing: 07.05.2025
(51) Int. Cl.: A61K 9/00, A61K 9/08, A61K 41/00, A61K 49/00

(54) **IODINE-FREE INDOCYANINE GREEN J-AGGREGATES**

(71) Applicant: Provepharm Life Solutions, 13013 Marseille (FR)
(72) Inventor: MEMBRAT, Romain, 13013 MARSEILLE (FR); SAYAH, Babak, 13013 MARSEILLE (FR); LAURENT, Marina, 13013 MARSEILLE (FR); HELIOT, Armelle, 13013 MARSEILLE (FR); PTCHELKINE, Denis, 13013 MARSEILLE (FR)
(74) Representative: Bandpay & Greuter

(57) **Abstract**

The present invention relates to a supramolecular structure formed by the self-assembly of iodine-free indocyanine green molecules and a salt of ascorbic acid, preferably sodium ascorbate, into J-type aggregates exhibiting a helical filamentous configuration. The invention further relates to pharmaceutical compositions comprising said supramolecular structures and to their applications in photodynamic therapy (PDT) and/or photothermal therapy (PTT).

## Description

### TECHNICAL FIELD

The present invention relates to a supramolecular structure comprising indocyanine green J-aggregates and to a method for their preparation. More specifically, the invention pertains to biocompatible, iodine-free formulations of indocyanine green J-aggregates stabilized by a salt of ascorbic acid, and their use in photothermal and/or photodynamic therapies.

### TECHNICAL BACKGROUND

Indocyanine green (ICG) is a fluorescent cyanine dye that has been widely used in the medical field for more than six decades. Approved by the FDA in 1959, ICG is primarily used for diagnostic and imaging purposes due to its unique optical properties, including near-infrared fluorescence. These properties enable it to penetrate biological tissues, making it an invaluable tool for visualizing vascular structures, tissue perfusion, and organ functionality.

Clinically, ICG is employed in a variety of applications, such as evaluating hepatic function, cardiac output, and retinal blood flow. It is also widely used in fluorescence angiography for ophthalmology and neurosurgery, as well as for tumor localization and lymphatic mapping.

Structurally, ICG consists of a polymethine backbone between two indole rings with sulfonate groups. This structure allows ICG to absorb light in the near-infrared region (approximately 780 nm) and emit fluorescence at longer wavelengths (around 805 nm).

The structure of ICG not only gives the dye its unique optical properties but also allows it, under certain conditions, to self-assemble into aggregates, such as dimers, H-aggregates, and J-aggregates. Among these, J-aggregates are particularly noteworthy due to their highly ordered arrangement and enhanced optical properties compared to monomeric ICG (Dai et al., Nanotheranostics 2017, Vol. 1, pages 430-439).

J-aggregates of ICG are supramolecular structures formed by the self-assembly of ICG molecules into head-to-tail configurations, stabilized by π-π interactions and electrostatic forces. This ordered molecular arrangement causes a redshift in the UV-visible absorption spectrum to around 900 nm, enabling more effective absorption of near-infrared light. As a result, J-aggregates exhibit enhanced photothermal conversion, the process of transforming absorbed light into heat, which is crucial for applications like photothermal therapy in cancer treatment. Additionally, the J-aggregated form improves resistance to photobleaching, a degradation process where prolonged light exposure leads to the loss of fluorescence and therapeutic efficacy. In monomeric ICG, photobleaching often occurs due to covalent dimerization under irradiation, but this is significantly reduced in J-aggregated structures. These properties make J-aggregates good candidates for advanced biomedical imaging and therapeutic applications, in particular in photothermal therapies.

Despite their promising properties, the practical application of ICG J-aggregates is hindered by several significant challenges. One major limitation lies in the difficulty of precisely controlling their synthesis, particularly in terms of aggregate size, structural symmetry, and stability in solution and in vivo. Moreover, the detailed structural characteristics of these aggregates remain poorly understood.

Achieving defect-free and reproducible self-assembly of ICG monomers into well-defined J-aggregates is especially challenging. To the best of the applicant's knowledge, there is currently no available technique that enables the formation of ICG J-aggregates in a manner that is simultaneously simple, rapid, and reproducible.

Some strategies have been proposed to address the challenges associated with the formation and stability of ICG J-aggregates. For example, liposome-templated J-aggregates have shown enhanced stability and improved biocompatibility, while other nanostructured carriers, such as polymersomes and nano-emulsions, have contributed to better structural integrity. However, these methods often involve complex, multi-step fabrication processes or rely on materials that are non-biodegradable, thereby limiting their pharmaceutical applicability and scalability for clinical applications. Additionally, the use of certain carriers may incur significant production costs.

Chemical modifications, including lipid bilayer-assisted aggregation, have also been employed to enhance photothermal performance. Yet, such modifications frequently fall short in addressing critical concerns related to toxicity, reproducibility, and industrial feasibility.

Stabilizing agents, such as liposomes, calixarenes, micelles, and chemotherapeutic agents like cisplatin, have been explored to preserve the structural integrity of J-aggregates, although concerns persist due to the high cytotoxicity of some agents, such as cisplatin. While these supports can be effective to some extent, they often introduce additional complexity to the synthesis process and may raise concerns regarding biocompatibility. Furthermore, their limited biological stability can compromise the consistency and reliability needed for the clinical applications of J-aggregates.

There is therefore a need for a simple, fast, and reproducible method to synthesize ICG J-aggregates with controlled properties, using biocompatible and clinically acceptable materials. Such a solution must overcome the limitations of current approaches in order to unlock the full therapeutic and diagnostic potential of J-aggregates in biomedical applications.

There is also a need for a methodology that allows precise control over the self-assembly of ICG into J-aggregates with defined and reproducible characteristics such as size, symmetry, spectral properties, and stability.

Moreover, although photothermal therapy (PTT) based on the use of near-infrared absorbing agents is a highly promising field, it remains largely in the experimental stages. To date, no photothermal therapy utilizing ICG J-aggregates has been approved for clinical use. Likewise, there are currently no commercially available pharmaceutical products based on J-aggregates of ICG. This further highlights the need for robust, scalable, and clinically translatable technologies to exploit the unique advantages of J-aggregates for therapeutic and diagnostic purposes.

### SUMMARY OF THE INVENTION

The present invention provides a supramolecular structure comprising:
a) a plurality of iodine-free indocyanine green molecules arranged in a head-to-tail stacked configuration forming J-type aggregates; and
b) molecules of at least one pharmaceutically acceptable salt of ascorbic acid associated with said indocyanine green molecules.

In one embodiment, the supramolecular structure is formed by a plurality of repeating molecular subunits, each repeating molecular subunit comprising at least two, preferably from 2 to 5, indocyanine green molecules, and at least three, preferably from 3 to 7 molecules of pharmaceutically acceptable salt of ascorbic acid.

Preferably, each repeating molecular subunit comprises 3 indocyanine green molecules and 5 molecules of pharmaceutically acceptable salt of ascorbic acid.

Preferably, the repeating molecular subunits do not comprise covalently bound indocyanine green dimers.

In one embodiment, the repeating molecular subunits form protofilaments.

In one embodiment, the protofilaments organize into a helical configuration, preferably the helix comprises from 2 to 6 protofilaments, preferably 4 protofilaments.

Preferably, the helical configuration is defined by a pitch ranging from 100 Å to 300 Å and/or a radius ranging from 25 Å to 50 Å, as determined by cryogenic transmission electron microscopy.

Preferably, the helical configuration exhibits C2 symmetry.

Preferably, each repeating molecular subunit has a helical rise in the range of 6.0 A to 7.5 Å, as determined by cryogenic transmission electron microscopy.

Preferably, each repeating molecular subunit has a twist angle in the range of 8° to 12°, as determined by cryogenic transmission electron microscopy.

Preferably, the molecules of the pharmaceutically acceptable salt of ascorbic acid are non-covalently bound with the iodine-free indocyanine green molecules and are aligned along the longitudinal axis of the protofilaments.

In one embodiment, the pharmaceutically acceptable salt of ascorbic acid is sodium ascorbate.

The present invention also provides a method for the preparation of a supramolecular structure according to the invention, the method comprising:
- providing iodine-free indocyanine green and at least one pharmaceutically acceptable salt of ascorbic acid;
- dissolving said indocyanine green and said salt of ascorbic acid in an aqueous medium.

Preferably the concentration of iodine-free indocyanine green in the aqueous medium ranges from 0.1 mg/mL to 50 mg/mL, preferably from 1 to 25 mg/mL, and the weight ratio of the pharmaceutically acceptable salt of ascorbic acid to indocyanine green in the aqueous solution ranges from 0.15 to 30.

The present invention also concerns a pharmaceutical composition comprising the supramolecular structure as defined above and in detail below, and a pharmaceutically acceptable medium, preferably selected from the group consisting of sterile water for injection, saline, phosphate-buffered saline and combinations thereof.

The present invention also concerns the supramolecular structure of the invention or the pharmaceutical composition comprising it for use in photothermal therapy, photodynamic therapy and/or photoacoustic therapy.

The present invention introduces a novel and highly controllable method for synthesizing J-aggregates of ICG, addressing long-standing challenges such as poor reproducibility, limited structural control, and concerns regarding biocompatibility and toxicity.

This invention offers a simple, scalable solution based on the use of iodine-free ICG and a salt of ascorbic acid, thereby eliminating allergenic risks associated with iodine-containing formulations.

The inventors have discovered that this combination enables the spontaneous and reproducible self-assembly of ICG into well-defined J-aggregates, which further organize into helical supramolecular structures.

The inventors have found that the presence of the salt of ascorbic acid, in conjunction with the absence of iodine, plays a critical role in both promoting the formation and enhancing the stability of the obtained ICG J-aggregates.

The helical assemblies exhibit robust structural integrity, with well-defined molecular geometry and thermodynamic stability compared to monomeric ICG and to prior art J-aggregates of ICG.

These helical assemblies also remain intact in aqueous environments without the need for additional stabilizers, in particular after long-term storage.

Other advantages of the invention also include:
- use of only pharmaceutically acceptable, biocompatible components;
- long-term structural and optical stability of the aggregates;
- enhanced optical performance relative to monomeric ICG and to prior art J-aggregates of ICG.

Accordingly, this invention provides an industrially scalable approach for the production of ICG J-aggregates with precisely defined structural and functional characteristics. This paves the way for the broader clinical adoption of ICG J-aggregates in diagnostic imaging, photothermal and photodynamic therapy, and other biomedical applications.

### BRIEF DESCRIPTION OF THE DRAWINGS

**Figure 1** shows the UV-visible absorption spectrum of a comparative formulation (Formulation F1) comprising indocyanine green, iodine, and sodium ascorbate with different dilution factors ranging from 10 to 400. The X-axis represents the wavelength in nanometers (nm), and the Y-axis shows absorbance in arbitrary units (AU).
**Figure 2** shows a UV-visible spectra of a composition according to the invention comprising iodine-free indocyanine green and sodium ascorbate (Formulation F2) with different dilution factors ranging from 10 to 400.
**Figure 3** shows the UV-visible spectra of a formulation prepared by reconstituting Infracyanine (from Serb) in water for injection with different dilution factors ranging from 20 to 400. Spectra were recorded at three time points: immediately after reconstitution (t₀), 3 hours post-reconstitution (t3h), and 6 hours post-reconstitution (t6h).
**Figure 4** shows the UV-visible spectra of a formulation obtained by reconstituting Diagnostic Green (from HUB) in water for injection with different dilution factors ranging from 20 to 400. Spectra were recorded at t₀ (immediately after reconstitution), t3h (3 hours), and t6h (6 hours).
**Figure 5** shows the UV-visible absorption spectra of Formulation F2 after the addition of 5% by weight of sodium iodide.
**Figure 6** shows the UV-visible absorption spectra of Formulation F2 (according to the invention) with varying concentrations of sodium ascorbate.
**Figure 7** shows comparative transmission electron microscopy images of: **(a)** Formulation F2 (iodine-free ICG with sodium ascorbate) at t=0; **(b)** Formulation F2 after 3 months of storage; **(c)** iodine-free ICG in the absence of sodium ascorbate at t=0; **(d)** Formulation F1 (comprising iodine and sodium ascorbate) after 3 months of storage; **(e)** reconstituted Infracyanine (Serb) immediately after reconstitution; and **(f)** reconstituted Diagnostic Green (HUB) immediately after reconstitution.
**Figure 8** shows transmission electron microscopy images illustrating the effect of sodium ascorbate concentration on Formulation F2: **(g)** Formulation F2 containing 70% of the nominal sodium ascorbate concentration; **(h)** Formulation F2 containing 50% of the nominal sodium ascorbate concentration; **(i)** Formulation F2 containing 15% of the nominal sodium ascorbate concentration.
**Figure 9** shows 2D class averages obtained by high-resolution cryo-TEM, of well-defined filamentous helical supramolecular structures within Formulation F2 according to the invention (iodine-free ICG with sodium ascorbate).

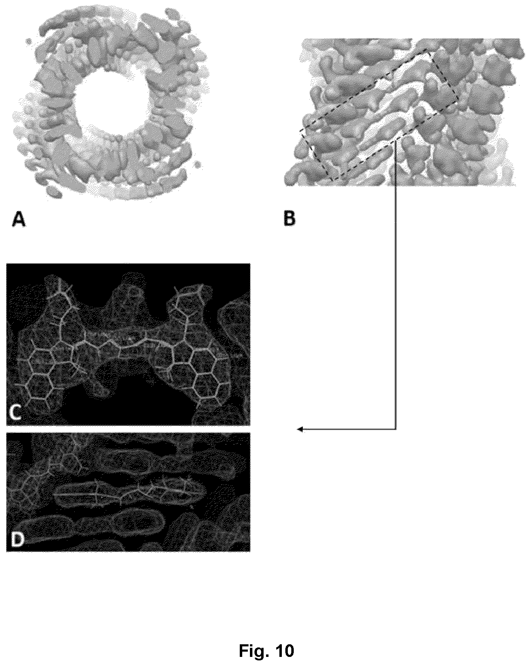

**Figure 10** shows the reconstruction of the molecular structure of the J-aggregates in Formulation F2 (iodine-free ICG with sodium ascorbate) from electron density mapping and molecular modeling. **(A)** shows a three-dimensional top-down view of the supramolecular structure with a helical, staircase-like stacking (J-type aggregates) of ICG molecules. **(B)** provides a lateral close-up of the helical stack. **(C)** shows a molecular-level view of two ICG molecules within the selected region in B. **(D)** presents a complementary side view showing the stacking interface of ICG molecules with sodium ascorbate molecules intercalated between them.
**Figure 11** shows the reconstructed helical molecular structure of the J-aggregates formed in Formulation F2 (iodine-free ICG with sodium ascorbate), showing the alignment and fitting between the electron density map **(A)** and the molecular model **(B).**

### DETAILED DESCRIPTION

The invention will now be described in more detail without limitation in the following description.

The expression "comprised between X and Y" includes boundaries, unless explicitly stated otherwise. This expression means that the target range includes the X and Y values, and all values from X to Y.

Throughout the description and claims of this specification, the words "comprise" and "contain" and variations of the words, for example "comprising" and "comprises", mean "including but not limited to", and do not exclude other moieties, additives, components, integers, or steps. Moreover, the singular encompasses the plural unless the context otherwise requires, in particular, where the indefinite article is used, the specification is to be understood as contemplating plurality as well as singularity, unless the context requires otherwise.

Where upper and lower limits are quoted for a property, for example for the concentration of a component, then a range of values defined by a combination of any of the upper limits with any of the lower limits may also be implied.

In one aspect, the present invention provides a method for preparing supramolecular J-type aggregates of indocyanine green (ICG) by combining iodine-free ICG with at least one pharmaceutically acceptable salt of ascorbic acid, preferably in an aqueous medium. This process results in the spontaneous self-assembly of the dye molecules into highly ordered, filamentous helical supramolecular structures, as further described below.

According to an embodiment, the method comprises the following steps:
- providing iodine-free ICG;
- providing at least one pharmaceutically acceptable salt of ascorbic acid, preferably sodium ascorbate;
- dissolving or dispersing both components in a pharmaceutically acceptable medium, preferably an aqueous medium.

The iodine-free ICG is preferably provided in high-purity form, either as a crystalline powder, lyophilized material, or pre-dissolved solution.

The pharmaceutically acceptable salt of ascorbic acid used in the context of the present invention may be employed in any suitable form, including but not limited to solid (e.g., powder or crystalline) form or pre-dissolved in an aqueous medium. The salt may be added directly to the formulation as a dry component or introduced as a solution prepared in advance.

The sequence of addition of the components is not critical to the formation of J-aggregates. For example, the ICG may be dissolved first, followed by the addition of the ascorbate salt, or vice versa.

In one embodiment, a portion of the ascorbate salt is first dissolved in the aqueous medium, and ICG is then introduced into the resulting solution. Following an initial period that allows for partial self-assembly, a second portion of sodium ascorbate, preferably pre-dissolved in water, may be added to the mixture.

The aqueous medium may be a pharmaceutically acceptable solvent, that is safe and nontoxic upon administration to humans or animals and is useful for the preparation of a pharmaceutical formulation.

The pharmaceutically acceptable aqueous medium used in the present invention may be any biologically acceptable liquid in which the components of the composition, particularly iodine-free indocyanine green and a pharmaceutically acceptable salt of ascorbic acid, are soluble or dispersible. Suitable aqueous media include, but are not limited to, water for injection (WFI), bacteriostatic water for injection (BWFI), sterile saline solution, Ringer's solution, dextrose solution, glucose solution, and phosphate-buffered saline (PBS).

The aqueous medium may optionally contain one or more pharmaceutically acceptable additives, such as buffers, bacteriostats, or solutes, to adjust pH or render the solution isotonic with blood, thereby enhancing compatibility for parenteral administration.

According to a preferred embodiment, the aqueous medium is selected from WFI or BWFI, with water for injection being especially preferred.

The molar or weight ratio of the ascorbate salt to ICG may vary depending on the salt used but should be sufficient to promote and stabilize J-aggregate formation. In preferred embodiments, the weight ratio of ascorbate salt to ICG ranges from 0.15 to 30, more preferably from 0.20 to 15, and most preferably from 0.20 to 13.

The concentration of ICG in the aqueous composition may range from 0.1 mg/mL to 50 mg/mL, preferably from 1 to 25 mg/mL, and most preferably from 1 to 10 mg/mL. The concentration of the ascorbate salt may range from 0.015 mg/mL to 1500 mg/mL, preferably from 0.2 to 750 mg/mL, and most preferably from 0.2 to 130 mg/mL.

The solution is optionally gently mixed to facilitate dissolution or dispersion of the components, preferably at room temperature, typically between 20°C and 25°C.

Preferably, no external energy input such as heating or sonication is required to initiate the aggregation process. In another aspect, the present invention also relates to the supramolecular structure obtained or obtainable by the method described herein.

In another aspect, the present invention also relates to a supramolecular structure as described in detail below.

### The iodine-free indocyanine green

For the purpose of the invention, the term indocyanine green or ICG refers to the compound with the formula sodium 4-[2-[(1E,3E,5E,7Z)-7-[1,1-dimethyl-3-(4-sulfonatobutyl)benzo[e]indol-2-ylidene]hepta-1,3,5-trienyl]-1,1-dimethylbenzo[e]indol-3-ium-3-yl]butane-1-sulfonate represented by formula (VII) below. The term indocyanine green may also encompass pharmaceutically acceptable salts, solvates, hydrates, acids, anhydrous forms, and free base forms of this compound.

As used herein, the term "iodine-free" refers to indocyanine green compositions that are substantially free of iodide ions or any iodine-containing species such as sodium iodide or any other iodinated compounds, whether as intentional excipients or process-related impurities.

Preferably, the iodine-free ICG used in the invention contains less than 0.01% by weight of iodide, and more preferably contains no detectable iodine, as measured by inductively coupled plasma mass spectrometry (ICP-MS).

Preferably, iodine-free indocyanine green used in the present invention is obtained by a method that includes at least one step of salification of indocyanine green in acid form using sodium chloride to produce the sodium salt of indocyanine green commonly designated as indocyanine green.

For the purpose of the invention, the term "indocyanine green in acid form" refers to the compound with the formula 4-[2-[7-[1,1-dimethyl-3-(4-sulfobutyl)benzo[e]indol-3-ium-2-yl]hepta-2,4,6-trienylidene]-1,1-dimethylbenzo[e]indol-3-yl]butane-1-sulfonic acid, which is represented by formula (VI) below.

In particular, the iodine-free ICG can be prepared by a method comprising at least a step d) of treating or reacting 4-[2-[7-[1,1-dimethyl-3-(4-sulfobutyl)benzo[e]indol-3-ium-2-yl] hepta-2,4,6-trienylidene]-1,1-dimethylbenzo[e]indol-3-yl]butane-1-sulfonic acid of formula (VI) with sodium chloride to obtain 3,3,3',3'-tetramethyl-1,1'-di(4-sulfobutyl)-4,5,4',5'-dibenzoindotricarbocyanine sodium salt of formula (VII) according to the following scheme:

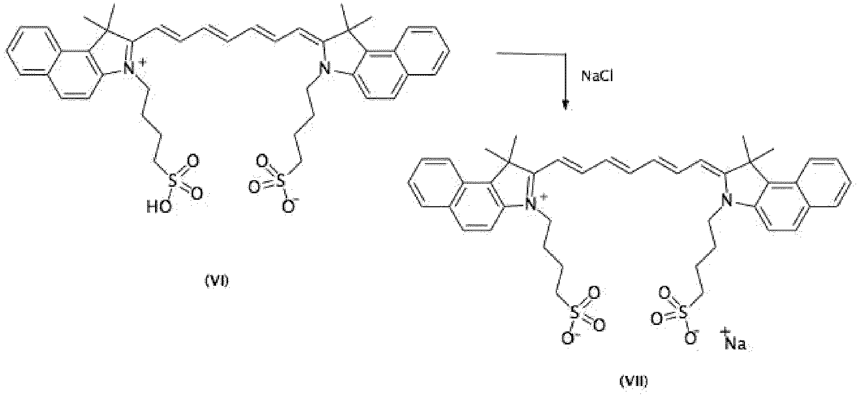

Preferably, in step d), sodium chloride is used in an amount such that the molar ratio indocyanine green in acid form of formula (VI) to sodium chloride is less than or equal to 1:1, preferably at a molar ratio ranging from 1:5 to 1:1, more preferably from 1:3 to 1:1.

Preferably, the reaction of step d) is carried out in a solvent chosen from the group consisting of water, methanol, ethanol, isopropylalcohol, n-propanol, n-butanol and mixtures thereof.

According to a favorite embodiment, step d) is carried out in a mixture of water and methanol. Preferably, water and methanol are mixed in a volume ratio ranging from 5:1 to 1:5, preferably from 2:1 to 1:5, more preferably from 2:1 to 1:2.

Advantageously, step d) is carried out at a temperature ranging from 25 to 65 °C, preferably from 40 to 55 °C.

Advantageously, after completion of the reaction of step d), the reaction mixture is cooled down to room temperature and the product of formula (VII) is isolated from the reaction medium by precipitation. Preferably, this precipitation is carried out by the addition of a solvent chosen from the group consisting of ethers, water, acetone and mixtures thereof.

According to an embodiment, the indocyanine green of formula (VII) is isolated from the reaction medium by precipitation with acetone.

Alternatively, it could be envisaged to isolate the product of formula (VII) from the reaction medium by other purification techniques such as solvent extraction or column chromatography.

The indocyanine green in acid form of formula (VI) may be obtained by any process known to the skilled person, such as for example the process described in WO2017/093889A1 or in US5,750,722.

According to a preferred embodiment, indocyanine green in acid form, represented by formula (VI), is prepared through a process comprising at least steps a), b), and c) as described below.

Step a) consists of reacting 1,2,2-trimethylbenzo[e]indole of formula (I) with 1,4-butanesultone of formula (II) in an equimolar ratio to obtain 4-(1,1,2-trimethyl-1H-benzo[e]indolium-3-yl)butane-1-sulfonate of formula (III) according to the following scheme:

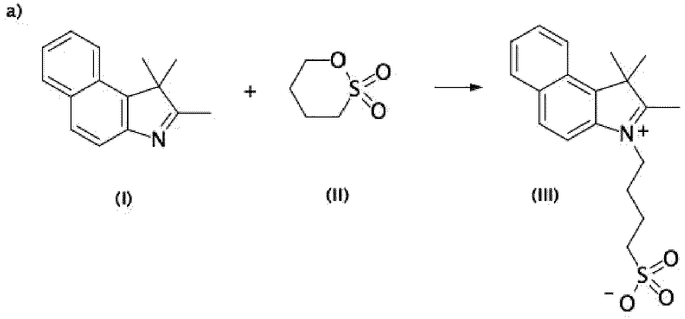

This reaction is known to the skilled person and is described, for example, in US5,750,722, WO2017/093889A1 and Chem. Eur. J. 2021, 27, 14535-14542.

The reaction of step a) may be carried out in a solvent chosen from the group consisting of pentane, hexane, heptane, cyclohexane, cyclopentane, petroleum ether, xylene, toluene, and mixtures thereof. According to a favorite embodiment, step a) is carried out in toluene.

Advantageously, the reaction of step a) is carried out at a temperature ranging from 100 °C to 180 °C, preferably from 120°C to 160°C, more preferably from 120°C to 150°C.

Advantageously, after completion of the reaction, the reaction mixture is cooled down to room temperature and the product of formula (III) is isolated from the reaction medium by precipitation. Preferably, this precipitation is carried out by the addition of a solvent chosen from the group consisting of ethers, water, acetone and mixtures thereof.

Step b) consists of condensing the intermediate of formula (III) obtained in step a) with glutaconaldehyde dianil hydrochloride of formula (IV) in an equimolar ratio in the presence of acetic anhydride to obtain 4-(1,1-dimethyl-2-((1E, 3E, 5E)-6-(N-phenyl acetamido)-hexa-1,3,5-trienyl)-1H-benzo[e]indolium-3-yl)-butane-sulfonate of formula (V) according to the following scheme:

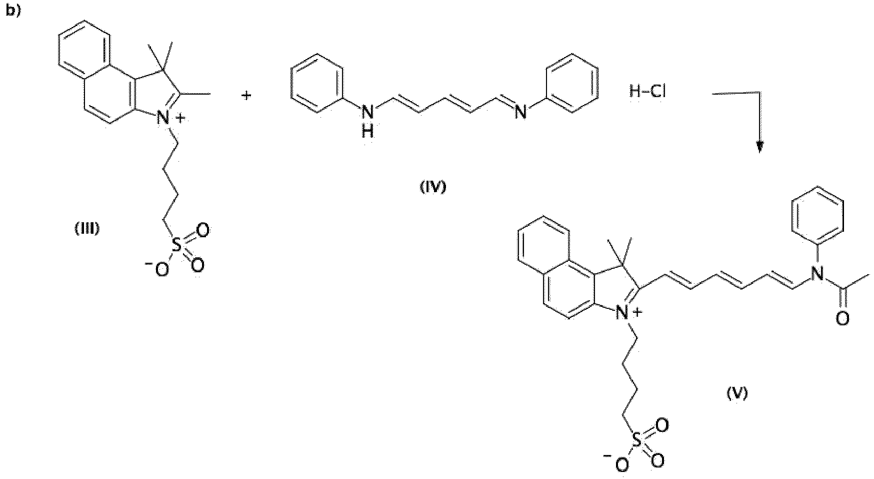

This reaction is described, for example, in EP2764861, US5,750,722 and US2019/0337896.

Alternatively, the reaction of step b) can be carried out in the presence of maleic anhydride instead of acetic anhydride.

Preferably, the reaction of step b) is carried out at a temperature ranging from 50°C to 170°C, preferably from 60°c to 150 °C, more preferably from 70°C to 130 °C.

Advantageously, after completion of the reaction, the reaction mixture is cooled down to room temperature and the product of formula (V) is isolated from the reaction medium by precipitation. Preferably, this precipitation is carried out by the addition of a solvent chosen from the group consisting of ethers, water, acetone and mixtures thereof.

Step c) consists of reacting the intermediate of formula (V) obtained in step b) with 4-(1,1,2-trimethyl-1H-benzo[e]indolium-3-yl)butane-1-sulfonate of formula (III) in an equimolar ratio in the presence of a base to obtain 4-[2-[7-[1,1-dimethyl-3-(4-sulfobutyl)benzo[e]indol-3-ium-2-yl]hepta-2,4,6-trienylidene]-1,1-dimethylbenzo[e]indol-3-yl]butane-1-sulfonic acid of formula (VI) according to the following scheme:

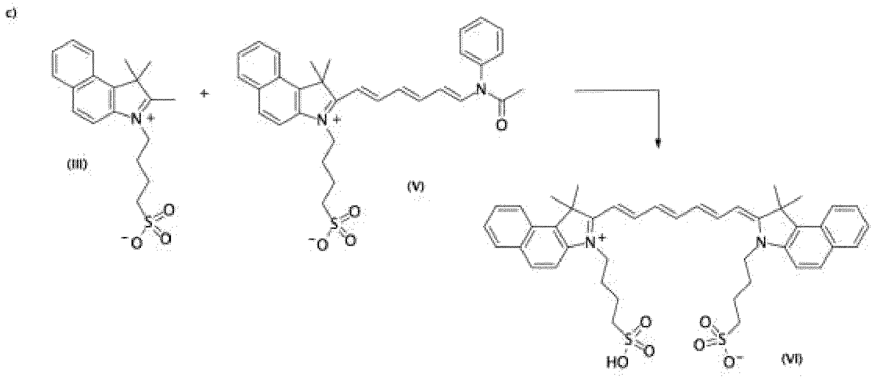

This reaction is described, for example, in EP2764861, US5,750,722 and US2019/0337896.

The reaction of step c) may be carried out in a solvent chosen from the group consisting of water, methanol, ethanol, isopropyl alcohol, propanol, butanol and mixtures thereof. According to a favorite embodiment, the reaction of step c) is carried out in a mixture of water and methanol. Preferably, water and methanol are mixed in a volume ratio ranging from 1:1 to 1:3.

Preferably, the base in step c) is chosen from a group consisting of triethylamine, pyridine, ammonia, sodium carbonate, sodium bicarbonate and sodium hydroxide. More preferably, the base is triethylamine.

Preferably, the reaction of step c) is carried out at a temperature ranging from 20 °C to 40 °C, preferably at 25°C.

The intermediates of formulae (III), (V) and (VI) can be purified after steps a), b) and c) respectively. The purification of these intermediates can be carried out by one or more conventional purification steps such as precipitation, crystallization, solvent extraction. The purification can be carried out, for example, according to the procedure disclosed in the examples of application US2019/0337896.

According to an embodiment, step d) of salification of the indocyanine green in acid form with sodium chloride can be followed a step e) of purification of the obtained indocyanine green.

According to a favorite embodiment, the purification step e) is carried out by recrystallization of indocyanine green of formula (VII).

Preferably, the purification step e) comprises at least the steps of:
e1) suspending, or dispersing, or dissolving, the indocyanine green of formula (VII) in a solvent,
e2) heating the composition obtained at step e1),
e3) cooling down the solution obtained in step e2),
e4) recovering indocyanine green in crystalline form.

Advantageously, the solvent in step e1) is chosen from the group consisting of water, acetone, methanol, ethanol, isopropyl alcohol, n-propanol, n-butanol and mixtures thereof.

Preferably, the solvent is step e1) is a mixture of water and acetone.

More preferably, in step e1), water and acetone are used in a ratio ranging from 10:1 to 1:10 v/v, preferably from 4:1 to 1:6 v/v, more preferably from 1:1 to 1:5 v/v.

Advantageously, where the composition obtained in step e1) is a suspension or a dispersion, this composition is heated in step e2) to a temperature allowing the solubilization of indocyanine green.

Advantageously, in step e2) the reaction medium is heated to a temperature superior or equal to 40 °C, preferably to a temperature ranging from 40 to 80 °C, preferably from 40°C to 65°C.

Advantageously, in step e3) the solution is cooled down or allowed to cool down to a temperature comprised between 10°C and 25°C, preferably between 15°C and 25°C.

By "cooled down", is meant that the reaction medium is left to cool down to ambient temperature, or alternately, that active cooling down is implemented, for example by immerging the reactor in a cooler environment than ambient temperature, in order to accelerate the drop in temperature.

According to a favorite embodiment, the solution is then maintained at this temperature for a sufficiently long time to form ICG crystals. Preferably, the solution is maintained at a temperature between 10 and 25°C, preferably between 15°C and 25°C for a period of time ranging from 10 to 120 hours, preferably from 24 to 96 hours, more preferably from 48 to 80 hours.

Optionally, the solution is then further cooled down to a temperature inferior or equal to 10°C, preferably at a temperature ranging from -5 and 10°C, more preferably from 0 and 10 °C.

According to a preferred embodiment, step e3) is carried out without stirring or agitating the solution.

By "step e3) is carried out without stirring or agitating the solution", it is meant that all sub-steps, i.e., cooling to a temperature comprised between 10 and 25°C, maintaining the solution at this temperature for a certain period of time, and optionally cooling to a temperature below 10°C, are carried out without stirring or agitating the reaction medium.

Advantageously, in step e4), crystalline indocyanine green is isolated from the reaction medium by filtration.

Advantageously, after step e4), crystalline indocyanine green is further purified by at least one step e5) of washing crystalline indocyanine green with a solvent.

Advantageously, washing is carried out with a solvent chosen from an acidic aqueous solution, acetone, acetonitrile and mixtures thereof.

Preferably, washing is carried out at least once with acetone and at least once with acetonitrile.

More preferably, washing is carried out once to three times with acetone and once to three times with acetonitrile.

The impurities contained in the indocyanine green can be any reaction byproduct or intermediate resulting from the process of manufacture. For example, as described in the document US2019/0337896, the impurities can include N-phenylacetamide, 4-(1,1,2-trimethyl-1H-benzo[e]indolium-3-yl) butane-1-sulfonate, 4-(1,1,2-dimethyl-2-((1E, 3E, 5E)-6-(N-phenylacetamido) hexa-1,3,5-trienyl)-1H-benzo[e]indolium-3-yl) butane-1-sulfonate, but are not limited thereto.

Preferably, the indocyanine green is provided as a composition comprising a content of metallic contaminants of less than 200 ppm, preferably, less than 100 ppm, more preferably less than 50 ppm, even more preferably less than 20 ppm, by weight of metals based on weight of indocyanine green. The metallic contaminant content in the indocyanine green can be measured by Inductively Coupled Plasma Mass Spectrometry (ICP-MS).

By "metallic contaminants" it is to be understood the so-called "heavy" metals and in particular: Al, As, Cd, Cr, Cu, Fe, Sn, Mn, Hg, Mo, Ni, Pb, Zn as well as their organic and inorganic derivatives.

Still more preferably, the indocyanine green comprises no or less than 2 ppm of lead and Arsenic.

The indocyanine green used in the composition of the invention is provided as a composition that does not comprise sodium iodide.

The indocyanine green used in the composition of the invention may be provided in any suitable form such as a lyophilizate or a crystalline powder.

### The pharmaceutically acceptable salt of ascorbic acid

The present invention involves the use of at least one pharmaceutically acceptable salt of ascorbic acid in combination with iodine-free indocyanine green to promote the formation and stabilization of J-type aggregates. The salt of ascorbic acid plays a dual role as a biocompatible excipient and a structural stabilizer, facilitating the spontaneous self-assembly of ICG molecules into supramolecular filaments with helical symmetry.

As used herein, the term "pharmaceutically acceptable salt of ascorbic acid" refers to any salt derived from ascorbic acid that is suitable for administration to humans or animals.

Non-limiting examples include sodium ascorbate, potassium ascorbate, calcium ascorbate, magnesium ascorbate, and any mixtures thereof.

According to a preferred embodiment, the pharmaceutically acceptable salt of ascorbic acid is sodium ascorbate, which may be derived from the D- or L-enantiomer of ascorbic acid, preferably from the L-enantiomer.

The amount of the ascorbate salt used in the composition may vary depending on the specific salt, but should be sufficient to induce and maintain J-aggregate formation under aqueous conditions.

In certain embodiments, the composition consists essentially of iodine-free indocyanine green and at least one pharmaceutically acceptable salt of ascorbic acid in a pharmaceutically acceptable medium, preferably aqueous medium.

In certain embodiments, the composition consists of iodine-free indocyanine green and at least one pharmaceutically acceptable salt of ascorbic acid in a pharmaceutically acceptable medium, preferably aqueous medium.

In one embodiment, the composition of the invention may further comprise one or more additional compounds selected from those known by the skilled person to be suitable for medical and/or diagnosis solutions.

When present, the additional compound may be selected, for example, from the group consisting of ethylenediaminetetraacetic acid (EDTA), D-alpha-tocopherol polyethylene glycol 1000 succinate (Vitamin E-TPGS), thiomalic acid, isosorbide, PEG 400, PEG 1000, thioglycolic acid, thiourea, alpha and beta cyclodextrin, thiolactic acid, glutathione reduced, thioglycerol, sorbitol, methionine, sodium chloride, histidine, dithiothreitol (DTT) and mixtures thereof.

Preferably, the additional compound is selected from glutathione reduced, thioglycerol, sorbitol, methionine, sodium chloride, histidine, dithiothreitol (DTT) and mixtures thereof. For example, the weight ratio of this additional compound to indocyanine green in the composition may range from 1:500 to 100:1, preferably from 1:500 to 10:1, more preferably from 1:100 to 10:1, most preferably from 1:100 to 4:1.

In a preferred embodiment, the composition is substantially free of surfactants, and more specifically of non-ionic surfactants.

In particular, the composition may comprise less than 0.1% by weight, preferably less than 0.05% by weight of surfactants, and most preferably is entirely free of surfactants (0% by weight).

### The supramolecular structure

According to another aspect, the present invention provides a supramolecular structure comprising:
a) a plurality of iodine-free indocyanine green molecules arranged in a head-to-tail π stacked configuration, forming J-type aggregates; and
b) molecules of at least one pharmaceutically acceptable salt of ascorbic acid associated with said indocyanine green molecules.

As used herein, the term "J-aggregates" or "J-type aggregates" refers to non-covalently bound, highly ordered supramolecular molecular assemblies of iodine -free ICG, wherein the molecules adopt a head-to-tail orientation stabilized by π-π stacking interactions.

As used herein, the term "head-to-tail orientation stabilized by π-π stacking interactions" refers to an ordered, non-covalent supramolecular arrangement in which a plurality of planar dye molecules, here iodine-free indocyanine green, are aligned such that the electron-rich π-systems of adjacent molecules interact in a directional fashion, with the electron-donating "tail" region of one molecule positioned adjacent to the electron-accepting "head" region of the next molecule along a defined stacking axis. Structurally, this head-to-tail arrangement contrasts with H-type (face-to-face) stacking, in which dye molecules align in a parallel, co-facial manner without the directional offset characteristic of J-aggregates.

As used herein, the term "associated" refers to a non-covalent interaction between the molecules of the pharmaceutically acceptable salt of ascorbic acid and the iodine-free indocyanine green molecules. This association is preferably formed by weak electrostatic interactions, including cation-π interactions, where the cationic species from the salt (e.g., sodium ions) interact with the electron-rich π-system of the ICG molecules. Additional polar, non-covalent forces, such as dipole-dipole interactions and electrostatic attractions, may also contribute to the stabilization of the supramolecular structure.

The Applicant has surprisingly found that the pharmaceutically acceptable salt of ascorbic acid, in particular sodium ascorbate, plays a critical role within the supramolecular structure by promoting and stabilizing the self-assembly of ICG molecules into J-type aggregates as described herein.

In one embodiment, the supramolecular structure comprises a plurality of repeating molecular subunits, each of which includes a combination of iodine-free indocyanine green molecules and a pharmaceutically acceptable salt of ascorbic acid.

According to an embodiment, each repeating subunit comprises at least 2 molecules of iodine-free ICG.

According to an embodiment, each repeating subunit comprises at most 5 molecules of iodine-free ICG.

According to an embodiment, each repeating subunit comprises at least one, preferably at least 3 molecules, preferably at least 4 molecules of pharmaceutically acceptable salt of ascorbic acid, preferably sodium ascorbate.

According to an embodiment, each repeating subunit comprises at most 8, preferably at most 7, more preferably at most 6 molecules of pharmaceutically acceptable salt of ascorbic acid, preferably sodium ascorbate.

In a preferred embodiment, each repeating subunit comprises from 2 to 5 molecules of iodine-free ICG and from 3 to 7 molecules of a pharmaceutically acceptable salt of ascorbic acid.

In a more preferred embodiment, each subunit comprises 3 molecules of iodine-free ICG and 5 molecules of a pharmaceutically acceptable salt of ascorbic acid, preferably sodium ascorbate.

According to a specific embodiment, the J-aggregates of the present invention are formed from individual iodine-free ICG monomers and do not comprise or originate from covalently bound ICG dimers. This structural distinction is critical, as it contributes to unique morphological properties of the aggregates.

This distinction is significant because J-aggregates formed from monomeric ICG units (non-covalently bound ICG molecules) according to the invention exhibit different supramolecular organization and spectroscopic behaviour compared to those originating from dimers.

According to a preferred embodiment, the repeating subunits form protofilaments.

According to a preferred embodiment, the protofilaments further organize into a helical configuration.

In a particularly preferred embodiment, each helical supramolecular structure is composed of from 2 to 6, preferably four protofilaments twisted together around a common axis to form an elongated filamentous helix.

Preferably, the resulting structure displays periodic organization and a defined helical pitch as described below.

The geometry of the helical configuration may be defined by:
- a helical pitch ranging from 100 Å to 300 Å, preferably from 120 Å to 260 Å, and more preferably about 129 Å; and/or
- a radius ranging from 25 Å to 50 Å, preferably about 35 Å.

As used herein, the term " helical pitch" refers to the axial distance along the central longitudinal axis of a helical supramolecular structure that is required for the helix to complete one full 360° rotation. In other words, the pitch is the linear distance measured parallel to the helix axis between two points on the helix that are in equivalent spatial orientation, such as the same side or face of a repeating molecular subunit, after one complete turn of the spiral. The pitch thus reflects the periodicity of the helical structure.

As used herein, the term "radius" refers to the perpendicular linear distance measured from the central longitudinal axis of a helical supramolecular structure to the outermost boundary or surface of the filament formed by the assembled molecules. This parameter defines the radial dimension of the helix and reflects the spatial extent of the supramolecular structure in the direction orthogonal to the helical axis. In the context of the present invention, the radius corresponds to half of the total helix diameter and represents the lateral distance occupied by the stacked molecular subunits.

The geometry of the helical configuration may also be defined by a repeat distance ranging from 100 Å to 300 Å, preferably from 120 Å to 260 Å, and more preferably about 129 Å.

The term "repeat distance", as used herein, refers to the axial length between two identical points on consecutive molecular subunits within a helical supramolecular structure. It describes how far along the axis you must go to find an identical molecular configuration. In helices with one subunit per turn, where each repeating unit completes a full 360° twist, the repeat distance equals the pitch. This type of arrangement is referred to as in-register stacking, which is characteristic of highly symmetric helical architectures.

In preferred embodiments, the supramolecular structure of the invention is characterized by a repeat distance equal to the helical pitch. This configuration corresponds to in-register stacking of the molecular subunits.

In preferred embodiments, the helical configuration exhibits rotational symmetry, preferably a C2 symmetry.

As used herein, the term "rotational symmetry," designated as Cn symmetry, refers to a structural feature of a supramolecular assembly whereby the structure maintains an indistinguishable appearance upon rotation around its central axis by an angle of 360°/n, where n is a positive integer. This means that the molecular arrangement repeats itself n times during a full 360° rotation, indicating that the structure possesses n-fold rotational symmetry. For example, a structure exhibiting C2 symmetry would appear identical every 180° of rotation around the helix axis.

Each repeating molecular subunit within the helix may be further defined by:
- a rise per unit between 6.0 Å and 7.5 Å, preferably about 6.8 Å; and/or
- a twist angle between 8° and 12°, preferably 9° to 10.5°.

As used herein, the term " rise per subunit" refers to the linear distance measured along the central helical axis, between two adjacent repeating molecular subunits. This parameter quantifies the incremental vertical translation that occurs as the structure progresses from one molecular unit to the next along the helix. In other words, the rise per subunit defines how much the structure advances upward or downward with each successive unit in the spiral arrangement.

As used herein, the term "twist angle" refers to the angular rotation between two successive repeating molecular subunits around the central helical axis of a supramolecular structure. It quantifies the degree to which each subunit is rotated relative to the preceding one as the helix progresses along its axis.

In a preferred embodiment, the molecules of the pharmaceutically acceptable salt of ascorbic acid, preferably sodium ascorbate, are non-covalently bound with the iodine-free indocyanine green molecules and are aligned along the longitudinal axis of the protofilaments forming the helical supramolecular structure. In this configuration, the sodium ascorbate molecules interact with the ICG monomers through non-covalent forces, such as cation-π, electrostatic, and dipole-dipole interactions, stabilizing the head-to-tail stacking of the ICG molecules.

The geometric parameters of the supramolecular structure as described above may be determined by any technique known in the field of structural molecular analysis. Preferably, the geometric parameters are determined by cryo-transmission electron microscopy (cryo-TEM) combined with Fourier-based image analysis and optionally three-dimensional reconstruction methods.

In particular, cryo-TEM enables direct visualization of the aggregates at near-atomic resolution under cryogenic conditions, preserving native morphology. The image data acquired may be subjected to a sequence of analytical steps including motion correction, contrast transfer function correction (to restore phase information), filament extraction, and 2D classification or class averaging (to improve signal-to-noise ratio and isolate representative structural features). Thereafter, power spectrum analysis of class averages or filament segments may be used to extract the helical symmetry parameters, such as pitch, twist angle, rise per subunit, and to identify any rotational symmetry (Cn symmetry). Electron density maps generated by 3D helical reconstruction can further confirm the presence of repeating subunits, and validate the supramolecular organization, including the arrangement of indocyanine green and associated ascorbate molecules.

Preferably, the supramolecular structure is further characterized by optical absorbance in the near-infrared region, preferably between 800 nm and 950 nm.

The pharmaceutically acceptable salt of ascorbic acid plays a critical role in the formation and stabilization of the supramolecular structure. Specifically, without being bound to any particular theory, it may serve to:
- facilitate the self-assembly of iodine-free indocyanine green molecules into ordered J-type aggregates;
- promote the helical organization of said aggregates into well-defined filamentous structures;
- enhance the thermodynamic stability of the supramolecular assembly in aqueous environments; and
- suppress or prevent the formation of disordered H-aggregates or amorphous precipitates.

Preferably, the supramolecular structure of the invention exhibits structural and functional stability over time following long-term storage. This stability may be defined by the preservation of key parameters such as aggregate morphology (e.g., fibrous helical structure), spectral features (e.g., near-infrared absorbance), and resistance to precipitation or disassembly.

In a preferred embodiment, the supramolecular structure remains stable for at least 1 month when stored under ambient conditions, typically between 20°C and 25°C, or under controlled refrigeration conditions such as from 2 to 8°C.

In a more preferred embodiment, said stability is maintained for at least 2 months, preferably for at least 3 months, more preferably for at least 6 months, and most preferably for at least 12 months under the same storage conditions showing no significant change in morphology or absorbance profile.

In a most preferred embodiment, the formulation remains stable for up to 2 years, whether stored under ambient temperature or under controlled refrigeration conditions such as from 2 to 8°C.

Stability may be assessed by periodic evaluation of the UV-visible absorption spectrum in the near-infrared region (preferably 800-950 nm), preferably combined with structural analysis techniques such as cryo-TEM to confirm the maintenance of aggregate size, shape, and order.

### Formulation forms

It is another aspect of the invention to provide a composition comprising the supramolecular structure described in detail above.

The supramolecular structure described above, is preferably obtained in a pharmaceutically acceptable medium, preferably an aqueous medium comprising iodine-free ICG and at least one pharmaceutically acceptable salt of ascorbic acid.

In one embodiment, the resulting aqueous solution is used directly as a ready-to-use composition, suitable for immediate administration and/or packaging.

Advantageously, the aqueous solution has an osmolarity of at least 1 mOsM. More advantageously, the aqueous solution has an osmolarity comprised between 1 and 300 mOsM, more preferably between1 and 200 mOsM.

Advantageously, the aqueous solution has a pH ranging from 5 to 7, preferably from 5.5 to 6.5.

In another embodiment, the solution undergoes lyophilization to provide a lyophilized solid composition, which can be reconstituted with an appropriate aqueous medium prior to use.

The terms "lyophilized solid composition" or "lyophilized powder" refers to the solid product obtained through lyophilization of the aqueous solution, comprising the J-aggregates described above. Lyophilization refers to a process in which the aqueous composition is first frozen, and the frozen solvent is subsequently removed by sublimation under vacuum.

When provided as a lyophilized powder, the composition can be reconstituted into a solution prior to administration using an aqueous medium, as previously described.

All limitations described above regarding the aqueous medium, concentrations and osmolarity also apply for the aqueous solution reconstituted from the lyophilized composition.

According to another aspect, the composition according to the invention may be provided in the form of a kit.

In one variant, the kit comprises a first compartment containing iodine -free indocyanine green and a salt of ascorbic acid in lyophilized form, and a second compartment, separate from the first, containing an aqueous medium.

In another variant, the kit comprises a first compartment containing iodine-free indocyanine green and a second compartment, separate from the first, containing an aqueous solution of a salt of ascorbic acid. In this variant, iodine-free indocyanine green is preferably provided in the form of a lyophilized powder.

The kits according to one or the other variant, are intended to be used to reconstitute extemporaneously an aqueous solution comprising J-aggregates resulting from the association of iodine-free indocyanine green and the salt of ascorbic acid.

The kits are preferably composed of parts in amounts such that the aqueous solution reconstituted from the kit has an ICG concentration ranging from 0.1 to 50 mg/ml, preferably from 0.1 to 30 mg/ml, more preferably comprised between 1 and 25 mg/ml, and most preferably between 1 and 10 mg/ml.

The kit can comprise two separate vials, packaged with or without a syringe, or it can comprise a pre-filed two-compartment syringe, like for example a dual-chamber by-pass syringe. Such a kit permits mixing the powder and the aqueous solution before administration.

### Medical and therapeutic uses

The supramolecular structures and pharmaceutical compositions described in the present invention are suitable for use in the medical, diagnostic, and therapeutic fields, in particular for applications involving light-activated therapies. These include, without limitation, photothermal therapy (PTT), photodynamic therapy (PDT) and photoacoustic (PA) treatment of tumors, wherein light energy is used to achieve selective cytotoxic or cytostatic effects in targeted tissues.

The supramolecular structure of the invention, comprising iodine-free indocyanine green J-aggregates stabilized by the pharmaceutically acceptable salt of ascorbic acid, exhibits enhanced near-infrared (NIR) absorbance, increased photostability, and well defined supramolecular geometry. These properties are advantageous in clinical applications requiring precise light activation, deep tissue penetration, and sustained functional integrity.

In some embodiments, the compositions of the invention comprising the supramolecular structure is used to treat malignant or non-malignant tumors, vascular abnormalities and/or localized infections.

In an embodiment, the composition is used in photothermal therapy, which is a minimally invasive treatment modality based on the conversion of NIR light into heat. Upon near-infrared irradiation, for example at a wavelength between 790 and 830 nm, preferably at 808 nm, the J-aggregates of the invention convert absorbed light into thermal energy, leading to localized heating and selective thermal damage of pathological tissue.

In one embodiment, the composition can be administered to a subject followed by targeted near-infrared irradiation at the target site, resulting in localized apoptosis or necrosis of tumor cells.

This photothermal effect may be used alone or in combination with other modalities, such as chemotherapy, radiotherapy, and/or immunotherapy.

Preferably, the composition is used for thermal ablation of solid tumors, including but not limited to cutaneous tumors, hepatic tumors, head and neck cancers, or deep-seated malignancies, by means of external or fiber-optic light delivery.

In some embodiments, the photothermal effect may be used in combination with real-time imaging based on the fluorescence of the J-aggregates of indocyanine green, allowing for visualization and monitoring of treatment response.

The composition may be administered via intratumoral, intravenous, intraarterial, or any other route that allows sufficient accumulation at the tumor site to achieve a photothermal effect. Preferably, the composition is administered via intratumoral injection.

In another embodiment, the composition is used in photodynamic therapy, wherein the J-aggregates of the composition serve as photosensitizers that generate reactive oxygen species, particularly singlet oxygen, upon exposure to NIR light. The generated reactive oxygen species induce oxidative stress, leading to cell death via apoptosis, necrosis, or autophagy.

The composition may be administered via intratumoral, intravenous, intraarterial, or any other route that enables sufficient accumulation at the tumor site to induce a photodynamic effect upon light activation.

In one embodiment, the composition is administered via intratumoral injection, followed by near-infrared exposure to activate the J-aggregates and elicit a photodynamic response. The staircase-like supramolecular stacking and enhanced NIR absorbance of the J-aggregates significantly improve photosensitization efficiency compared to monomeric indocyanine green.

In some embodiments, the photodynamic therapy method is applied to tumor margins during surgery, superficial lesions, and/or deep tissues accessed via minimally invasive techniques, such as endoscopic or fiber-optic probes.

In certain embodiments, the photothermal and photodynamic uses described above may be combined.

In certain embodiments, the composition of the invention is used in photoacoustic tumor applications, where pulsed NIR light is absorbed by the J-aggregates of the invention, leading to thermoelastic expansion and the generation of acoustic waves detectable by ultrasound transducers. This photoacoustic response enables deep-tissue imaging of tumors and can be used to guide or monitor therapy in real time.

In another aspect, the present invention relates to a method of treating a subject in need thereof, comprising administering to said subject a composition comprising iodine-free indocyanine green and a pharmaceutically acceptable salt of ascorbic acid, preferably sodium ascorbate, wherein said composition comprises supramolecular J-type aggregates as described herein, and subjecting the target site to near-infrared light to induce a therapeutic effect.

In certain embodiments, the method is a photothermal therapy method.

In other embodiments, the method is a photodynamic therapy method.

In still other embodiments, the method comprises a combination of photothermal therapy and photodynamic therapy.

According to one embodiment, the invention provides a method of treating a tumor in a subject, comprising:
i. administering to the subject a composition comprising J-aggregates of iodine-free indocyanine green and a pharmaceutically acceptable salt of ascorbic acid, preferably sodium ascorbate, as described herein;
ii. optionally, allowing the composition to accumulate at the tumor site;
iii. exposing the target tissue site to near-infrared light, preferably in the wavelength range of 790 nm to 830 nm, preferably at 808 nm;
wherein the absorbed light energy is converted into heat by the J-type aggregates of iodine-free indocyanine green and pharmaceutically acceptable salt of ascorbic acid, preferably sodium ascorbate, resulting in thermal ablation of tumor tissue.

In some embodiments, the tumor is a solid tumor, preferably selected from liver, breast, pancreatic, skin, bladder, or brain tumors.

Preferably, the pharmaceutical composition is administered by intratumoral injection.

Preferably, the near-infrared light is directed toward the tumor site via external laser irradiation for superficial or accessible tumors and/or via interstitial fiber-optic devices for deep-seated tumors.

In an embodiment, the method is performed under real-time image guidance, preferably using fluorescence imaging derived from the intrinsic optical properties of the indocyanine green, to enhance targeting precision.

In another embodiment, the invention provides a method of photodynamic therapy in a subject in need thereof, comprising:
i. administering to the subject a composition comprising J-aggregates of iodine-free indocyanine green and a pharmaceutically acceptable salt of ascorbic acid, preferably sodium ascorbate, as described herein;
ii. irradiating the target site with near-infrared light, preferably in the wavelength range of 790 nm to 830 nm, preferably at 808 nm,
   wherein the J-aggregates absorb the near-infrared light and generate reactive oxygen species, which induce localized oxidative cytotoxicity at the irradiated site.

In a variant, the composition is administered by intratumoral injection.

Preferably, the method is used to treat cancer, including early-stage lesions, cutaneous tumors, tumor margins and/or localized metastases. The method may be applied to internal tumors using endoscopic or catheter-based illumination systems.

In a variant, the method includes simultaneous or sequential photothermal and photodynamic therapy.

In another embodiment, the present invention provides a method for photoacoustic imaging and/or treatment of a subject in need thereof, comprising:
i. administering to the subject a composition comprising supramolecular J-type aggregates formed from iodine-free indocyanine green and a pharmaceutically acceptable salt of ascorbic acid, as described herein;
ii. irradiating the target tissue site with near-infrared light, preferably within the wavelength range of 700 nm to 900 nm, preferably from 780 to 810 nm;
iii. detecting the generated acoustic signals, preferably using an ultrasound transducer, and optionally;
iv. performing a therapeutic intervention guided by or in conjunction with the photoacoustic signal.

In another embodiment, the method further comprises applying concurrent or sequential therapeutic modalities, such as photothermal or photodynamic therapy, wherein the same irradiation used for photoacoustic excitation also induces a therapeutic effect.

In all embodiments, the administration route, dosage, and irradiation protocol may be adapted to the anatomical location of the target, tumor type, and desired therapeutic outcome. Such parameters may be determined and adjusted by a healthcare professional based on the subject conditions and the established medical practice in the field of oncology.

In some embodiments, the composition can be administered to patients with known iodine sensitivity and/or at risk of contrast-related adverse effects, as the formulation is free of iodine or iodide-containing excipients.

In further embodiments, the composition of the invention comprising J-aggregates of iodine-free ICG and a pharmaceutically acceptable salt of ascorbic acid, preferably sodium ascorbate, is suitable for repeated administration, including long treatment cycles or combined diagnostic/therapeutic protocols.

According to a further aspect, the invention provides the use of a composition comprising iodine-free indocyanine green and a pharmaceutically acceptable salt of ascorbic acid, preferably sodium ascorbate, as described herein in the manufacture of a medicament for use in treating tumors by photothermal therapy, treating tumors by photodynamic therapy or a combination of photothermal therapy and photodynamic therapy. In some embodiments, the medicament is further adapted for use in photoacoustic imaging and therapy.

### EXAMPLES

### I- Synthesis of indocyanine green

### ICG1 (comparative)

ICG1 was synthesized following the procedure described in WO201093889A1 by reacting 1,2,2-trimethylbenz[e]indole with 1,4-butanesultone and glutaconaldehyde dianile chlorhydrate. Sodium iodide was employed during the salification and purification steps. The final product was isolated as an amorphous green powder containing 3 to 4% by weight of iodine ions (expressed as Nal).

### ICG2 (iodine-free)

ICG2 was synthesized according to WO2023213805A1 by reacting 1,2,2-trimethylbenz[e]indole with 1,4-butanesultone and glutaconaldehyde dianile chlorhydrate. During the salification step, one molar equivalent of sodium chloride (NaCl) was added. The product obtained was a crystalline golden-red powder containing about 1% by weight of sodium chloride and no detectable iodine as determined by ICP-MS.

### II- Formulation preparation

### Formulation F1 (comparative)

A 500 mL double-jacketed glass reactor was charged with 500 mL of Milli-Q water (mQ). The solution was degassed with nitrogen for 1 hour at room temperature; an additional 10 mL of water was added to compensate for evaporation during degassing. Sodium ascorbate (500 mg) was rapidly introduced via a funnel, and the solution was further degassed with nitrogen for an additional 30 minutes at room temperature. After adjusting the nitrogen flush tube to the solution surface ("nitrogen sky"), ICG1 (1250 mg) was rapidly added to the reactor. The reaction mixture was stirred under nitrogen for 3 hours. The resulting bulk solution was subsequently filled into 2 mL DIN brown glass type I ampules over a period of 45 minutes.

### Formulation F2

A 500 mL double-jacketed glass reactor was charged with 480 mL of Milli-Q water containing 75 mg sodium ascorbate. An addition funnel containing a separately prepared solution of sodium ascorbate (425 mg in 30 mL water) was attached to the reactor. Both solutions were degassed separately under nitrogen atmosphere for 1 hour at room temperature with stirring at 150 RPM. After degassing, the nitrogen flush tube was positioned at the solution surface. ICG2 (1279 mg) was rapidly added to the reactor under stirring conditions (150 RPM). The mixture was stirred continuously under nitrogen for 7 hours at room temperature. Subsequently, the entire content of the addition funnel (30 mL sodium ascorbate solution) was introduced into the reactor, and stirring continued at 150 RPM for an additional 16 hours. The resulting bulk solution was filled into a 2 mL DIN brown glass type I ampules over a period of 45 minutes.

### III- Physico-chemical characterization

Physico-chemical properties of formulations F1 and F2 were compared against commercially available lyophilized ICG kits: Diagnostic Green from HUB and Infracyanine from Serb. Parameters evaluated included iodine content, pH, and osmolarity.

**Table 1**

| | **HUB** | **Serb** | **F1** | **F2** |
|---|---|---|---|---|
| **Presentation** | Vial of lyophilized ICG 25 mg | Vial of lyophilized ICG 25 mg | Ready to use solution in 10 mL ampule | Ready to use solution in 10 mL ampule |
| | Vial of ppi water 10 mL | Ampule of G5 solution 10 mL | | |
| **ICG concentration** | 2.5 mg/mL | 2.5 mg/mL | 2.5 mg/mL | 2.5 mg/mL |
| **Iodine content** | Yes <5% | 0 | Yes <5% | 0 |
| **Sodium ascorbate concentration** | - | - | 1 mg/mL | 1 mg/mL |
| **pH** | 6.2 | 5.3 | 6.8 | 6.15 |
| **Osmolarity** | 2 mOsm/L | 260 mOsm/L | 14 mOsm/L | 13 mOsm/L |

### IV- Spectroscopic analyses

Ultraviolet-visible (UV-vis) spectroscopy was employed to analyze and characterize the aggregation behavior of the prepared ICG formulations.

This technique enables the identification of different aggregation states, such as H-aggregates, J-aggregates, and monomeric forms, based on their distinct absorption characteristics. Specifically, H-aggregates are associated with a blue shift (absorption peaks at shorter wavelengths), while J-aggregates produce a red shift (absorption peaks at longer wavelengths) in the UV-vis spectra. These spectral shifts reflect differences in intermolecular interactions and molecular stacking arrangements within the formulations.

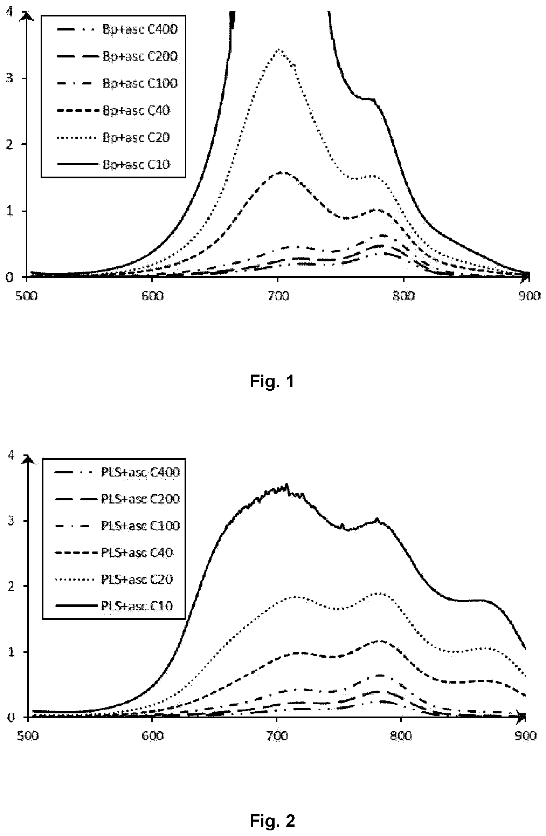

**Figure 1** shows the UV-visible absorption spectrum of Formulation F1, which contains ICG, iodine, and sodium ascorbate. The spectrum reveals characteristic absorption features including a blue-shifted band typical of H-aggregate formation (approximatively at 700 nm) and monomeric ICG (approximatively 780 nm),.

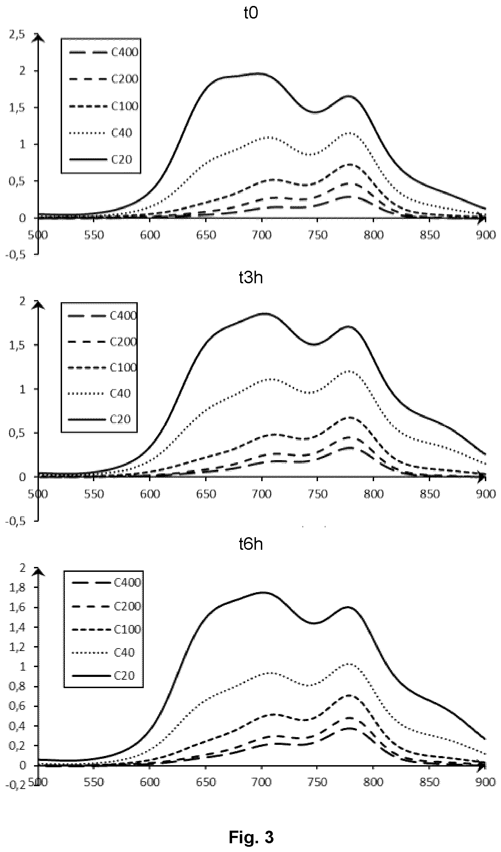

**Figure 2** presents the UV-visible spectrum of Formulation F2, comprising iodine-free ICG and sodium ascorbate. Unlike formulation F1, formulation F2 exhibits an additional absorption peak approximatively at 860-900 nm, which is indicative of J-aggregate formation. These J-aggregates differ from those reported in the literature, for example in Dai et al., Nanotheranostics 2017, Vol. 1, pages 430-439, by (i) a slightly higher absorbance maximum, and (ii) a broader, more irregularly shaped ("potato-like") absorption band.**Figure 3** shows the UV-visible spectra of a formulation prepared by reconstituting Infracyanine (Serb) in water for injection, recorded at three time points: immediately after reconstitution (t₀), and at 3 and 6 hours post-reconstitution (t₃h and t₆h). Across all time points, the formulation displays spectral features characteristic of H-aggregates and monomers, similar to F1. A distinct absorption band at approximately 650 nm is also present.

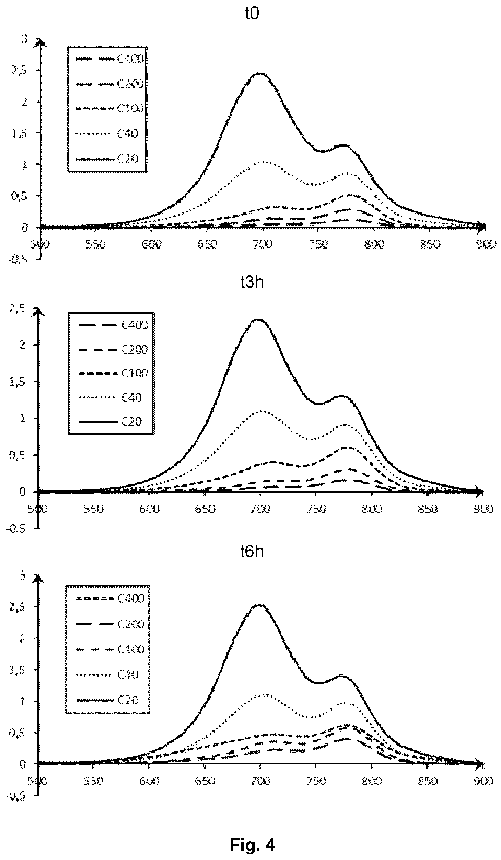

**Figure 4** illustrates the UV-visible spectra of Diagnostic Green reconstituted in water for injection, also recorded at t₀, t₃h, and t₆h. The spectra consistently demonstrate H-aggregate and monomeric absorption characteristics, with no significant spectral shifts or emerging peaks over time.

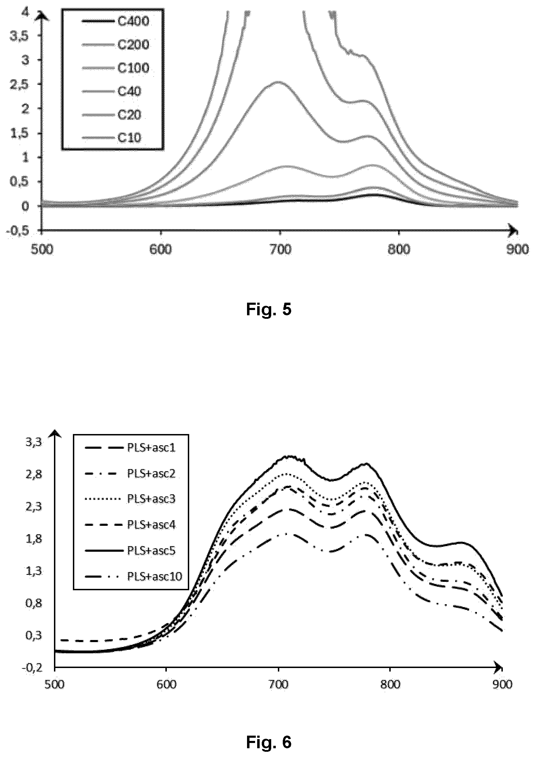

The role of iodide ions in modulating aggregation was investigated by supplementing the F2 formulation with 5% (w/w) sodium iodide (Nal). As shown in **Figure 5****,** this addition disrupted J-aggregate formation, as evidenced by the diminished intensity of the ~900 nm peak. This suggests that iodide interferes with the molecular organization required for J-aggregate assembly.

The effect of sodium ascorbate and ICG concentrations on J-aggregate formation was further explored in **Figure 6****.** UV-vis spectra were collected for the F2 formulation across five different sodium ascorbate concentrations: Asc 1 (1 mg/mL), Asc 2 (2 mg/mL), Asc 3 (3 mg/mL), Asc 5 (5 mg/mL), and Asc 10 (10 mg/mL). The results showed a clear concentration-dependent increase in the intensity of the J-aggregate peak, indicating that higher levels of sodium ascorbate (and ICG) promote more extensive J-aggregate formation.

### V- Transmission Electron Microscopy (TEM)

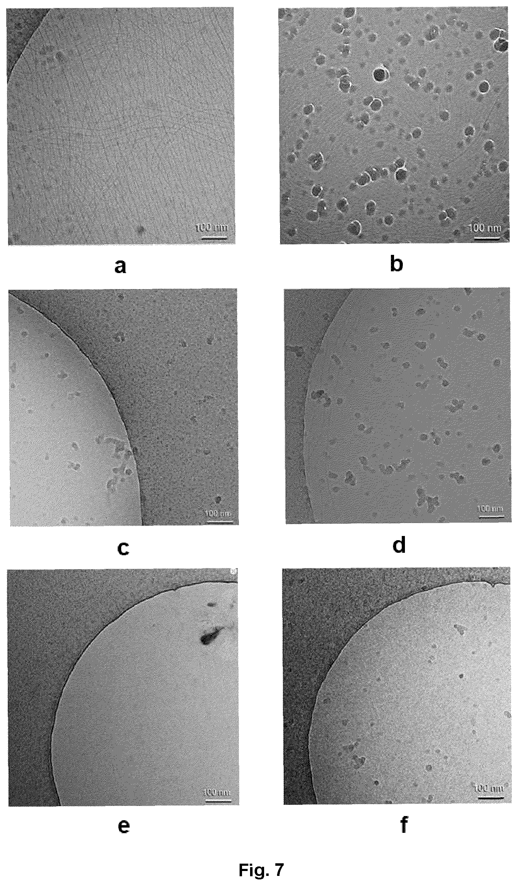

TEM was employed to directly visualize the morphology and nanoscale organization of ICG aggregates in Formulations F1 and F2, as well as in selected commercial ICG products. The TEM images in **Figure 7** reveal distinct differences in aggregate structure and density between the formulations, particularly in relation to the presence or absence of iodine and sodium ascorbate.

As shown in **Figure 7a****,** Formulation F2 (iodine-free ICG with sodium ascorbate) exhibited abundant, well-defined fibrous aggregates immediately after manufacturing (t = 0), indicating rapid and robust fiber formation. After three months of storage at ambient temperature **(****Figure 7b****),** these fibrous structures were still present, with only a minor decrease in fiber density, suggesting good long-term structural stability. In contrast, iodine-free ICG without sodium ascorbate **(****Figure 7c****)** showed sparse and poorly developed fibers, emphasizing the critical role of sodium ascorbate in promoting aggregate assembly.

Formulation F1, which contains both iodine and sodium ascorbate, showed fewer and less prominent fibers after three months of storage **(****Figure 7d****)** compared to Formulation F2, indicating that iodine likely disrupts or inhibits the formation of fibrous aggregates. Among commercial products, the Serb formulation (Infracyanine), reconstituted in water **(****Figure 7e****),** exhibited small, fragmented fibrous structures, suggesting a limited capacity for organized aggregation. Diagnostic Green (HUB) showed no detectable fibers **(****Figure 7f****),** consistent with a predominantly monomeric or H-aggregated state as indicated by UV-vis spectroscopy.

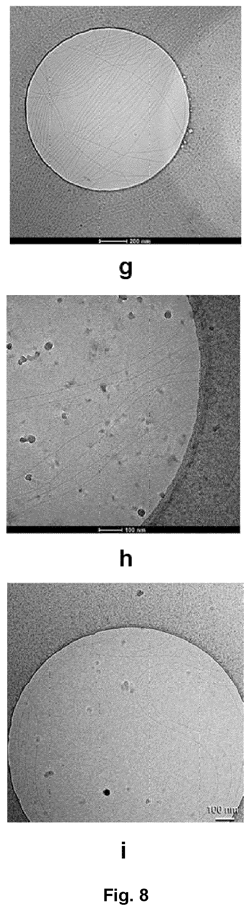

Further insight into the role of sodium ascorbate concentration is provided in **Figure 8****,** which presents TEM images of Formulation F2 prepared with decreasing ascorbate levels. When the formulation contained 70% of the nominal sodium ascorbate concentration, fibrous structures were still visible, though reduced in density **(****Figure 8g****).** At 50% concentration, fiber formation was noticeably diminished **(****Figure 8h****),** and at 15% concentration, fibers were virtually absent, with only scattered amorphous features observed **(****Figure 8i****).**

### VI- Structural characterization by cryogenic transmission electron microscopy (cryo-TEM)

To elucidate the structural organization of ICG aggregates formed by formulation F2, a detailed structural characterization was performed using cryo-TEM. The following sequential operations were carried out to process and analyze the acquired cryo-TEM data:
- Motion correlation: correction of specimen drift and beam-induced motion to enhance image clarity,
- Contrast transfer function correction, filament and particle selection, and particle extraction: improvement of image contrast and resolution, followed by precise selection and extraction of individual filaments and particles from the cryo-TEM micrographs,
- Determination of 2D class averages and helical symmetry assessment: classification of extracted particles into distinct two-dimensional classes to evaluate structural homogeneity and symmetry characteristics.

This analysis confirmed the presence of clear helical symmetry in the observed aggregates. Initial data processing allowed extraction of preliminary structural parameters, summarized as follows:
- Tube diameter: 100 Å
- Angular search range: 6°
- Helical rise: 10 Å

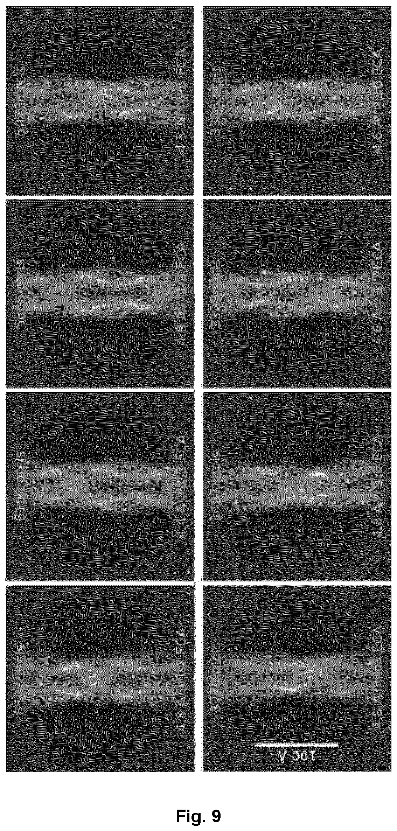

These preliminary observations established a foundation for subsequent1 high-resolution analyses. New TEM grids were prepared from Formulation F2 and examined using a higher-performance transmission electron microscope (Glacios). This advanced instrumentation enabled significantly enhanced resolution, allowing for clearer visualization of fine structural details and symmetry features. High-resolution imaging revealed distinct left-right symmetrical patterns, consistent with Cn symmetry, where n is an even integer. Representative 2D class averages illustrating these symmetrical features are shown in **Figure 9****.**

### VII- Determination of helical parameters via power spectrum analysis

To quantitatively determine the helical parameters of the observed ICG aggregates, power spectrum analyses were performed on cryo-TEM images obtained from formulation F2. This enabled the determination of the following preliminary helical parameters:
- Repeat distance: 129 Å
- Pitch (P): 129 Å
- Rise per subunit (p): 6.8 Å
- Radius (r): 35 Å

Calculated theoretical spectra based on these initial parameters were systematically compared with the experimentally derived spectra. This comparative analysis yielded three potential correlation solutions (Solutions 1, 2, and 3), each characterized by distinct sets of refined helical parameters as summarized in Table 2.

**Table 2**

| | **Calculated parameter** | **Solution 1** | **Solution 2** | **Solution 3** |
|---|---|---|---|---|
| **Pitch (P)** | 129 | 258.31 | 258.43 | 259.53 |
| **Rise (p)** | 6.8 | 6.20 | 6.85 | 7.33 |
| **Unit by laps (u)** | - | 40 | 38 | 36 |
| **Twist (t)** | - | 8.57 | 9.49 | 10.11 |
| **Radius** | 35 | 34 | 34 | 34 |

Electron density maps were subsequently generated for each of these three candidate solutions. Visual inspection and structural evaluation of these maps indicated that Solutions 1 and 3 exhibited continuous electron density between adjacent subunits, suggesting structural inconsistencies and unrealistic continuity patterns incompatible with the expected discrete helical organization.

In contrast, Solution 2 demonstrated clearly defined individual subunits without undesirable continuity artifacts between neighboring units. Therefore, Solution 2 was selected as the optimal structural model to accurately represent the helical organization of ICG aggregates in formulation F2. This selected solution served as a robust basis for identifying the precise helical subunit arrangement of the supramolecular structure.

### VIII- Molecular modeling

This step aimed at fitting a molecular model into the electron density map derived from the previously selected Solution 2.

Molecular modeling enabled precise identification and positioning of individual molecules within the electron density map. Analysis revealed that each repeating helical subunit contains three distinct molecules of ICG. As illustrated in **Figure 10** (dotted line), these ICG molecules exhibit a characteristic staircase stacking arrangement, indicative of J-type aggregation. This observation clearly confirms that the primary driving force behind the aggregation phenomenon is the formation of J-aggregates.

However, the overall structural organization proved to be more complex than a simple stacking of ICG molecules alone. Indeed, additional molecular components were identified within each helical subunit. Notably, sodium ascorbate molecules were found to form a central stacking arrangement located at the core of the helical aggregates. This central sodium ascorbate stacking plays an essential role in maintaining both the symmetry and structural stability of the aggregates.

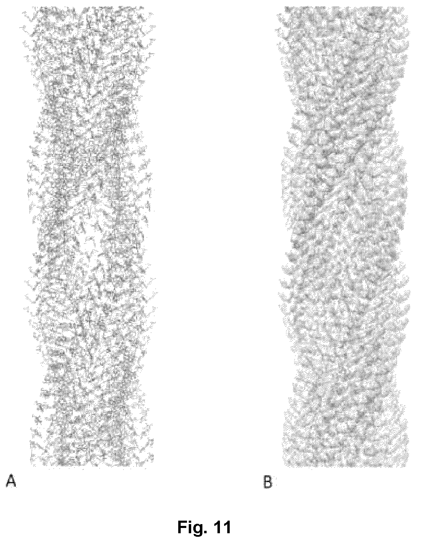

Based on these detailed modeling results, it was possible to precisely define the molecular composition of a single helical aggregate subunit and subsequently reconstruct the complete molecular organization of the filament. A high-quality fit between the proposed molecular model and the experimentally derived electron density map was achieved, as clearly demonstrated in **Figure 11****.**

### IX- Conclusion

The results confirmed that iodine-free indocyanine green in the presence of sodium ascorbate, undergoes a spontaneous self-assembly process forming J-type aggregates. This aggregation is notably inhibited by the presence of iodine. The resulting supramolecular structures are elongated fibers exhibiting well-defined helical symmetry, with all structural parameters precisely determined.

UV-visible spectroscopy confirmed the formation of J-aggregates, with a distinct absorption peak in the 850-950 nm range, indicative of head-to-tail molecular stacking.

Transmission electron microscopy revealed dense and persistent fibrous assemblies.

Cryo-TEM and power spectrum analysis established the helical parameters of the fibers, including a pitch of approximately 258 Å, a rise per subunit of about 6.8 Å, and a radius of 34-35 Å.

Molecular modeling and density fitting corroborated the stoichiometry and structural role of sodium ascorbate, which was identified as a central stabilizing element within the helical core.

## Claims

1. A supramolecular structure comprising:
(a) a plurality of iodine-free indocyanine green molecules arranged in a head-to-tail stacked configuration forming J-type aggregates; and
(b) molecules of at least one pharmaceutically acceptable salt of ascorbic acid associated with said indocyanine green molecules.

2. The supramolecular structure according to claim 1, comprising a plurality of repeating molecular subunits, each repeating molecular subunit comprising at least two, preferably from 2 to 5, indocyanine green molecules, and at least three, preferably from 3 to 7 molecules of pharmaceutically acceptable salt of ascorbic acid.

3. The supramolecular structure according to claim 2, wherein each repeating molecular subunit comprises 3 indocyanine green molecules and 5 molecules of pharmaceutically acceptable salt of ascorbic acid.

4. The supramolecular structure according to claim 2 or claim 3, wherein the repeating molecular subunits do not comprise covalently bound indocyanine green dimers.

5. The supramolecular structure according to any one of claims 2 to 4, wherein the repeating molecular subunits form protofilaments.

6. The supramolecular structure according to claim 5, wherein said protofilaments organize into a helical configuration, preferably the helix comprises from 2 to 6 protofilaments, preferably 4 protofilaments.

7. The supramolecular structure according to claim 6, wherein the helical configuration is defined by a pitch ranging from 100 Å to 300 Å and/or a radius ranging from 25 Å to 50 Å, as determined by cryogenic transmission electron microscopy.

8. The supramolecular structure according to claim 6 or 7, wherein the helical configuration exhibits C2 symmetry.

9. The supramolecular structure according to any one of claims 6 to 8, wherein each repeating molecular subunit has a helical rise in the range of 6.0 Å to 7.5 Å, as determined by cryogenic transmission electron microscopy.

10. The supramolecular structure according to any one of claims 6 to 9, wherein each repeating molecular subunit has a twist angle in the range of 8° to 12°, as determined by cryogenic transmission electron microscopy.

11. The supramolecular structure according to any one of claims 5 to 10, wherein the molecules of the pharmaceutically acceptable salt of ascorbic acid are non-covalently bound with the iodine-free indocyanine green molecules and are aligned along the longitudinal axis of the protofilaments.

12. The supramolecular structure according to any one of the preceding claims, wherein the pharmaceutically acceptable salt of ascorbic acid is sodium ascorbate.

13. Method for the preparation of a supramolecular structure according to any one of claims 1 to 12, comprising:
- providing iodine-free indocyanine green and at least one pharmaceutically acceptable salt of ascorbic acid;
- dissolving said indocyanine green and said salt of ascorbic acid in an aqueous medium.

14. The method according to claim 12, wherein the concentration of iodine-free indocyanine green in the aqueous medium ranges from 0.1 mg/mL to 50 mg/mL, preferably from 1 to 25 mg/mL, and the weight ratio of the pharmaceutically acceptable salt of ascorbic acid to indocyanine green in the aqueous solution ranges from 0.15 to 30.

15. A pharmaceutical composition comprising the supramolecular structure according to any one of claims 1 to 11 and a pharmaceutically acceptable medium, preferably selected from the group consisting of sterile water for injection, saline, phosphate-buffered saline and combinations thereof.

16. The composition according to any one of claims 1 to 11 for use in photothermal therapy, photodynamic therapy and/or photoacoustic therapy.
